(19) Europäisches Patentamt
European Patent Office
Office européen des brevets

(11) Publication number: 0 446 809 A1

# (12) EUROPEAN PATENT APPLICATION

(21) Application number: 91103588.9

(51) Int. Cl.[5]: C07D 307/80, C07D 307/85

(22) Date of filing: 08.03.91

(30) Priority: 12.03.90 IT 1964490

(43) Date of publication of application:
18.09.91 Bulletin 91/38

(84) Designated Contracting States:
BE CH DE ES FR GB LI SE

(71) Applicant: BIOMEDICA FOSCAMA INDUSTRIA CHIMICO-FARMACEUTICA S.P.A.
Via Tiburtina km 14,500
I-00131 Roma(IT)

(72) Inventor: Ceccarelli, Stefano
Via Dante Alighieri, 7
Frosinone(IT)
Inventor: Zanarella, Sergio
Via degli Abeti
Mentata, Rome(IT)
Inventor: De Vellis, Patrizia
Via Lago Maggiore 88
Frosinone(IT)
Inventor: Scuri, Romolo
Via Fosse Ardeatine, 234
Frosinone(IT)

(74) Representative: La Ciura, Salvatore
c/o STUDIO D'ORIO Via F. Sforza, 3
I-20122 Milan(IT)

## (54) New process for the synthesis of 5-acyloxy and 5-alkoxy-2,3-dihydro-4,6,7-trimethyl-2-(RS)-benzofuranacetic acids.

(57) The invention described here is a new process for the synthesis of 5-acyloxy and 5-alkoxy - 2,3 - dihydro - 4,6,7 - trimethyl - 2 - (RS) - benzofuranacetic acids, which are obtained through a smaller number of reactions, and whose unit costs are therefore lower making them more profitable.

EP 0 446 809 A1

The invention described here is a new process for the .. synthesis of 5-acyloxy and 5-alkoxy - 2,3 - dihydro - 4,6,7 - trimethyl - 2 - (RS) - benzofuranacetic acids.

In Italian patent application n° 21603 A/88 dated August 1989, there were claims for new dihydrobenzofuranic structures in whose general formula (I):

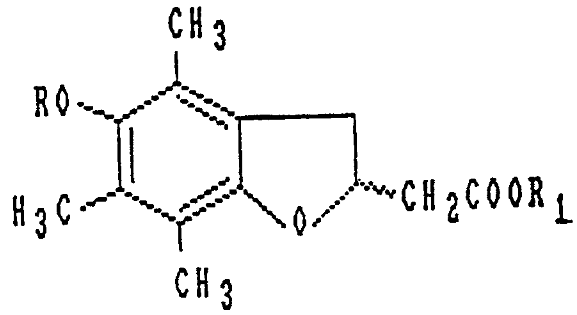

(I)

R is a hydrogen, an akyl of a mono or bicarboxylic acid, and a lower alkyl;

R1 is a hydrogen, an alkyl, an ether alkyl, an alkylamine, and an etherocyclic alkyl.

4,6,7 - trimethyl - 2 - (RS) - benzofuranacetic acid and 2,3 - dihydro - 5 - methoxy - 4,6,7 - trimethyl - 2 - (RS) - benzofuranacetic acid, have been shown to be effective in the treatment of many kinds of inflammation of the mucous .. membranes in the respiratory system, with a considerable antioxidant and radical-scavenger action.

The invention described here is a new process for the synthesis of 2,3 - dihydro - 4,6,7 - trimethyl - 2 - (RS) - benzofuranacetic acid, which is hereafter indicated by the abbreviation IRFI 005, and 5-acyloxy and 5-alkoxy - 2,3 -dihydro - 4,6,7 - trimethyl - 2 - (RS) - benzofuranacetic acids in whose general formula (II):

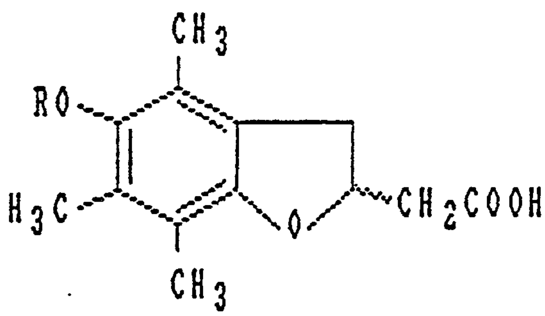

(II)

R is:

- a hydrogen;
- a -COR2 acyl, in which R2 is a linear or branched alkyl in which the number of carbon atoms varies between 1 and 4;
- a linear or branched alkyl in which the number of carbon atoms varies between 1 and 4.

The new process, in respect of the previously mentioned one, is characterized by the fact that the substrates are more easily accessible, and that fewer production stages are required, and consequently the product is easier to produce and more profitable. A number of toxic reagents and catalysts have also been eliminated from the process, making it much safer. The entire process ias also easier to reproduce, making it easier to extend it on an industrial scale.

The process described here consists of the reaction between derivates of 4-(2,5-dihydroxy-3,4,6-trimethylphenyl)-2-butanoic acid (especially its methyl ester), whose formula is (III).

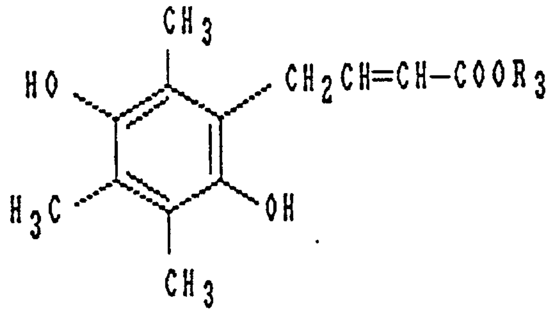

(III)

(In which R3 = hydrogen or lower alkyl, especially methyl), and an aqueous solution of an alkaline hydroxide such as KOH in the presence of a reducing agent such as Na2S204 at an adequate temperature (typically the solution reflux temperature), for a time of from 1 to 12 hours. The compound whose formula is (III) can be prepared in the way described by Mhorner and A.Nissen in DE-05-3.010.474 (1st October 1981), using a Friedel-Crafts alkylation reaction between trimethylhydroquinone and a suitable 2 or 3-butanoic acid derivate such as methyl 4-bromo-2-butanoate, in the presence of one or more inorganic acid catalysts. The compound (III) can be used in its raw state, the way it as after being produced by the alkylation reaction, for the cyclization reaction, thereby avoiding its isolation and purification. It can therefore also be mixed with some organic products such as trimethylhydroquinone and inorganic products such as zinc chloride.

The 2,3 - dihydro - 5 - hydroxy - 4,6,7 - trimethyl - 2 - (RS) - benzofuranacetic acid produced by the hydrolytic cyclization reaction is isolated by acidifying the reaction mixture, and then extracting it using an organic solvent. The product can therefore be separated from its non-acid impurities by extracting its sodium salt with an aqueous solution of sodium bicarbonate, or by crystallizing one of its salts using an organic base such as dicyclohexilamine. The IRFI 005 undisassociated form is made to precipitate in an aqueous acid environment.

The phenol group in the IRFI 005 compound is acylated by dispersing the 2,3 - dihydro - 5 - hydroxy - 4,6,7 - trimethyl - 2 - (RS) - benzofuranacetic acid in a suitable quantity of the anhydride of the acid (especially the acetic anhydride), with a quantity of a strong mineral acid (such as sulphuric acid) acting as a catalyst after being diluted with an inert solvent. The reaction mixture is maintained at a suitable temperature (normally between 60° and 90° C) for a time of from 0.5 to 12 hours. After being diluted with water, the 2,3 - dihydro - 5 -acyloxy - 4,6,7 - trimethyl - 2 - (RS) - benzofuranacetic acid precipitates, and can be purified by crystallization using a suitable solvent or mixture of solvents and treated with one of the normal de-coloring agents.

The alkylization of the phenolic hydroxyl is carried out by dissolving the IRFI 005 in an aqueous solution of an alkaline hydroxide, and gradually adding a suitable dialkyl sulphate (especially dimethyl sulphate). When the reaction is finished, the 2,3 - dihydro - 5 - alkoxy - 4,6,7 - trimethyl - 2 - (RS) - benzofuranacetic acid is obtained after the mixture has been acidified. Alternatively, a two-stage process can be used which consists of:

a) The formation of a 2,3 - dihydro - 5 - alkoxy - 4,6,7 - trimethyl - 2 - (RS) - benzofuranacetic alkyl by reacting the IRFI 005 compound with an excess of a suitable alkyl halide (especially methyl iodide) in a solution with an organic solvent such as dimethylformamide and in the presence of an acidity acceptor such as potassium carbonate;

b) The hydrolysis of the alkyl ester thus obtained to 2,3 - dihydro - 5 - alkoxy - 4,6,7 - trimethyl - 2 - (RS) - benzofuranacetic acid, by treating it with an alkaline hydroxide in water plus an organic solution.

The product is purified by crystallization using a suitable solvent or mixture of solvents and treated with one of the normal de-coloring agents.

The following examples illustrate the invention without limiting it.

EXAMPLE 1

2,3 - dihydro - 5 - hydroxy - 4,6,7 - trimethyl - 2 - (RS) - benzofuranacetic acid (IRFI 005).

The solvent is removed from a mixture of 152g of trimethylhydroquinone, 23g of zinc chloride anhydride, and 100ml of methyl 4 - bromo - 2 - butanoate (trans and cis) in 1 litre of n-heptane, after reaction for 2 hours at reflux temperature in an inert atmosphere has taken place. The raw residue is treated with 2.2 litres of a 20% solution of potassium hydrate containing 146g of sodium hyposulphite, and the

resuting mixture is heated to reflux temperature for 3 hours in an inert atmosphere. After cooling, a solution of hydrochloric acid at up to ph=3 is added, and the mixture is then extracted using ethyl acetate. after washing the organic extracts in a solution of sodium chloride, they are treated with a saturated aqueous solution of sodium bicarbonate. The aqueous solution containing the product is then concentrated and hydrochloric acid is added until the IRFI 005 compound has completely precipitated. The product (126g) can then be filtered and dried by crystallization with n-hexane/ethyl acetate. P.f. = 176-7°C; IR (KBr): 3374 (ν OH), 1705 cm-1 (ν COOH); 1H-NMR (DMSO-d6): δ 12.2 (1H,sb), 7.4 (1H,sb), 5.35-4.85 (1H;m), 3.5-2.8 (2H,m), 2.75-2.55 (2H,d), 2.1 (6H,s) 2.0 (3H,s). Elementary analysis of C13H1604 (P.M. 236.27): calc. C% 66.09, H% 6.83, found C% 66.16, H% 6.91.

EXAMPLE 2

2,3 - dihydro - 5 - acetoxy - 4,6,7 - trimethyl - 2 - (RS) - benzofuranacetic acid.

A solution of 0.1ml of concentrated sulphuric acid in 5ml of acetic acid is added, a little at a time, to a mixture of 20g of 2,3 - dihydro - 5 - hydroxy - 4,6,7 - trimethyl - 2 - (RS) - benzofuranacetic acid and 47ml of acetic anhydride. The resulting solution is heated to 80°C for 4 hours in an inert atmosphere. After cooling and the gradual addition of water, the product precipitates, and is then filtered and vacuum dried. It is purified using vegetable carbon as a de-coloring agent followed by crystallization using toluene. 16.8g of solid white crystals are obtained. P.f. = 167-8°C; IR (KBr): 1752 (ν AcO), 1710 cm-1 (ν COOH); 1H-NMR (CDCl3): δ 10.3 (1H,s), 5.3-4.8 (1H;m), 3.6-2.7 (4H,m), 2.3 (3H,s), 2.1 (3H,s) 2.0 (6H,s). Elementary analysis of C15H1805 (P.M. 278.30): calc. C% 64.74, H% 6.52, found C% 64.74, H% 6.90.

EXAMPLE 3

2,3 - dihydro - 5 - methoxy - 4,6,7 - trimethyl - 2 - (RS) - benzofuranacetic acid.

3.45g of potassium carbonate and 1.5ml of methyl iodide are added to a solution of 2.0g of 2,3 - dihydro - 5 - hydroxy - 4,6,7 - trimethyl - 2 - (RS) - benzofuranacetic acid in 15ml of dimethylformamide. The reaction mixture is continually agitated and kept at room temperature in an inert atmosphere for 24 hours, and then diluted with water and extracted with ethyl acetate. The organic solution is evaporated and vacuum dried, thus obtaining 2.4g of raw methyl 2,3 - dihydro - 5 - hydroxy - 4,6,7 - trimethyl - 2 - (RS) - benzofuranacetate (IR/ 1740 cm-1). This product is then dissolved in 10ml of acetone and treated with 34ml of 1N sodium hydroxide solution for 1 hour at reflux temperature. The solid which precipitates after the mixture has been cooled and acidified, is filtered, vacuum dried, treated with de-coloring carbon, and crystallized by benzene/n-hexane, thus obtaining 1.8g of solid white crystals. P.f. = 135-6°C; IR (KBr): 1702 cm-1 (ν COOH); 1H-NMR (CDCl3): δ 10.2 (1H,s), 5.2-4.8 (1H;m), 3.6 (3H,s), 3.4-2.5 (4H,m), 2.1 (6H,s), 2.05 (3H,s). Elementary analysis of C14H1804 (P.M. 250.29): calc. C% 67.18, H% 7.25, found C% 67.41, H% 7.42.

## Claims

1. Process for the preparation of 2,3 - dihydro - 5 - hydroxy - 4,6,7 - trimethyl - 2 - (RS) - benzofuranacetic acid, consisting of the reaction between one or more derivates of 4-(2,5-dihydroxy-3,4,6-trimethylphenyl)-2-butanoic acid, whose general formula is (III).

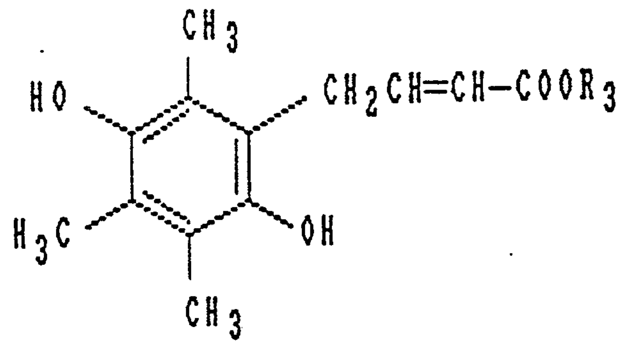

(III)

(In which R3 = hydrogen or lower alkyl, especially methyl; the double binding configuration can be either trans or cis), and an aqueous solution of a hydroxide of an alkaline metal in the presence of a reducing agent such as sodium hyposulphite.

**2.** Process for the preparation of 2,3 - dihydro - 5 - hydroxy - 4,6,7 - trimethyl - 2 - (RS) - benzofuranacetic acid as per Claim 1, characterized by the fact that the reaction takes place within the range of temperatures between room temperature and the reflux temperature of the solution described in Claim 1.

**3.** Process for the isolation and purification of 2,3 - dihydro - 5 - hydroxy - 4,6,7 - trimethyl - 2 - (RS) - benzofuranacetic acid obtained as per Claims 1 and 2, characterized by the fact that the reaction mixture is acidified, the product is extracted using an organic solvent such as ethyl acetate, and then salified with a suitable inorganic or organic base, and lastly the acid is once again isolated by being treated with an aqueous solution of a mineral acid followed by the filtering, drying, and the crystallization of the product which precipitates.

**4.** Process for the preparation of 2,3 - dihydro - 5 - acyloxy - 4,6,7 - trimethyl - 2 - (RS) - benzofuranacetic acids whose general formula is (II):

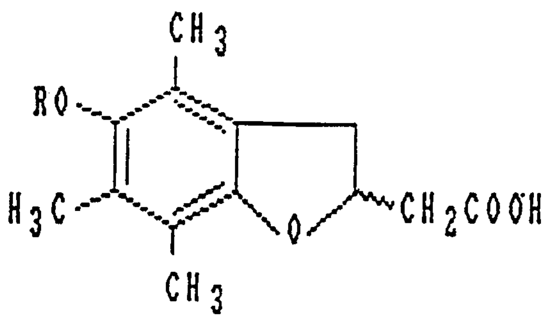

(II)

In which R is a linear or branched alkyl in which the number of carbon atoms can be from 2 to 5 (especially acetyl), consisting of the treatment of 2,3 - dihydro - 5 - hydroxy - 4,6,7 - trimethyl - 2 - (RS) - benzofuranacetic acid with a suitable anhydride of the acid in the presence of a quantity of a strong mineral acid, such as sulphuric acid, which acts as a catalyst and may be diluted with an inert solvent such as acetic acid.

**5.** Process for the preparation of 2,3 - dihydro - 5 - acyloxy - 4,6,7 - trimethyl - 2 - (RS) - benzofuranacetic acids as per Claim 4, characterized by the fact that the reaction takes place within the range of temperatures between room temperature and the reflux temperature of the solution described in Claim 4, and preferably between 40° and 90° C.

**6.** Process for the preparation of 2,3 - dihydro - 5 - alkoxy - 4,6,7 - trimethyl - 2 - (RS) - benzofuranacetic acids whose general formula is (II), in which R is a linear or branched alkyl in which the number of carbon atoms can be from 1 to 4 (especially methyl), consisting of the treatment of an aqueous alkaline solution of 2,3 - dihydro - 5 - hydroxy - 4,6,7 - trimethyl - 2 - (RS) - benzofuranacetic acid with a suitable dialkyl sulphate.

**7.** Two stage process for the preparation of 2,3 - dihydro - 5 - alkoxy - 4,6,7 - trimethyl - 2 - (RS) - benzofuranacetic acids as per Claim 6, consisting of the treatment of 2,3 - dihydro - 5 - hydroxy - 4,6,7 - trimethyl - 2 - (RS) - benzofuranacetic acid with an excess of an alkyl halide (especially methyl iodide) in a solution with an organic solvent such as dimethylformamide and in the presence of an acidity acceptor such as potassium carbonate, followed by the hydrolysis of the alkyl thus obtained, 2,3 - dihydro - 5 - alkoxy - 4,6,7 - trimethyl - 2 - (RS) - benzofuranacetate, by treating it with an alkaline hydroxide in a solvent consisting of water plus an organic solvent.

**8.** Process for the preparation of 2,3 - dihydro - 5 - alkoxy - 4,6,7 - trimethyl - 2 - (RS) - benzofuranacetic acids as per Claim 6 or 7, characterized by the fact that the reactions take place at a temperature in the

range from 0° to 100° C.

European Patent Office

# EUROPEAN SEARCH REPORT

Application Number

EP 91103588.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | DE - A1 - 3 925 496 (BIOMEDIA) * Claim 1; examples 1,2 * -- | 1-8 | C 07 D 307/80<br>C 07 D 307/85 |
| Y | CHEMICAL ABSTRACTS, vol. 102, No. 23, June 10, 1985, Columbus, Ohio, USA YOSHII, EIICHI et al. "Introduction of a 3-alkoxy-carbonyl-2-propenyl group at the ortho position of phenol and naphthol via alpha-aryl-oxy-gamma-butyrolactone. Application to synthesis of (±)-nanaomycin A and a 1-anthracenone" page 582, column 1, abstract -No. 203 787j & Chem. Pharm. Bull. 1984, 32(12),4779-85 -- | 1-8 | |
| | | | TECHNICAL FIELDS SEARCHED (Int. Cl.5) |
| A | CHEMICAL ABSTRACTS, vol. 110, No. 9, February 27, 1989, Columbus, Ohio, USA EJIRI, KATSUJI et al. "Dihydrobenzofuran derivatives as antioxidants and drug intermediates" page 630, column 1, abstract -No. 75 294x & Jpn. Kokai Tokkyo Koho JP 63 88,173 (88 88,173) -- | 1 | C 07 D 307/00 |
| A | US - A - 4 745 127 (ATKINSON) * Abstract * -- | 1 | |
| A | DE - A1 - 3 317 884 (HOECHST) * Page 9, lines 25-36 * ---- | 1 | |

The present search report has been drawn up for all claims

| Place of search | Date of completion of the search | Examiner |
|---|---|---|
| VIENNA | 02-05-1991 | HAMMER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document